# EUROPEAN PATENT SPECIFICATION

(11) **EP 4 391 945 B1**
(45) Date of publication and mention of the grant of the patent: **27.05.2026**
(21) Application number: 22761899.8
(22) Date of filing: 04.08.2022
(51) Int. Cl.: A61B 18/22, A61B 18/00, A61B 18/20

(54) **MULTI-LASER MEDICAL SYSTEM**
MEDIZINISCHES MULTILASERSYSTEM
SYSTÈME MÉDICAL MULTI-LASER

(30) Priority: 26.08.2021 US 202163237222 P
(43) Date of publication of application: 03.07.2024
(73) Proprietor: Boston Scientific Scimed, Inc., Maple Grove, MN 55311-1566 (US)
(72) Inventor: HASENBERG, Thomas, Charles, Campbell, CA 95008 (US); PENG, Steven, Yihlih, Fremont, CA 94536 (US); XUAN, Rongwei, Jason, Fremont, CA 94538 (US); YANG, Baocheng, Fremont, CA 94536 (US)
(74) Representative: Pfenning, Meinig & Partner mbB
(86) International application number: PCT/US2022/074505
(87) International publication number: WO 2023/028414

(56) References cited:
- US-A1- 2016 135 891
- US-A1- 2018 344 405
- US-A1- 2020 000 522
- US-A1- 2021 038 304

## Description

### TECHNICAL FIELD

This disclosure relates generally to medical/surgical laser systems, and more particularly, to medical laser systems including two or more laser devices, each laser device outputting a laser energy at a different wavelength for performing a medical procedure.

### BACKGROUND

Medical laser systems are used for a variety of surgical procedures. These procedures may include dusting and/or fragmentation of stones in the kidney, the bladder, and/or the ureter, and/or may be used for ablation, cauterization, or other medical procedures. The effectiveness of the medical procedure may be based on the absorption of laser energy by stones and other tissues, and the absorption of laser energy by stones and tissue may be based on the wavelength of the laser energy. Thus, it is necessary to provide a medical laser system capable of performing procedures on multiple different stone and/or tissue types.

Conventional medical laser systems include a single laser source configured to generate a laser energy having a single wavelength. One problem with conventional laser systems is that the wavelength of the laser energy should match, or be very close to, a peak optical absorption of the target tissue, e.g., a stone. If the wavelength of the laser energy is not close to the peak optical absorption of the target tissue, a higher laser power density is required to achieve the same temperature increase as a laser energy having a wavelength close to the peak optical absorption of the target tissue. Since the temperature increase of the tissue affects the efficiency of the ablation, dusting, or fragmentation, higher laser power densities are required if the wavelength of the laser energy does not match the peak optical absorption of the target tissue. This increased power density may increase the time and/or cost of the medical procedure, which may increase the risks involved with the medical procedure, including the risk of accidents or other complications. This disclosure may solve one or more of these problems or other problems in the art. The scope of the disclosure, however, is defined by the attached claims and not the ability to solve a specific problem.

US 2018/344405 teaches a system according to the preamble of present claim 1.

### SUMMARY OF THE DISCLOSURE

The present invention is defined in claim 1.

According to an aspect, a laser system includes a first laser configured to generate a laser light, wherein the first laser light includes a first wavelength, a second laser configured to generate a second laser light, wherein the second laser light includes a second wavelength different from the first wavelength, and a laser fiber operatively coupled to each of the first laser and the second laser configured to emit at least one of the first laser light or the second laser light at a target site.

The first laser light and the second laser light are emitted from the laser fiber based on a target tissue at the target site.

The first laser may further include a Thulium-doped fiber, and wherein the second laser may include a YAG laser rod co-doped with Holmium and Thulium.

The laser system may further include a third laser configured to generate a third laser light, wherein the third laser light may include a third wavelength different from the first wavelength and the second wavelength.

The third laser may include a Thulium-doped fiber.

The third wavelength may be approximately 2120 nm.

The laser system further includes a dedetector which is configured to detect a target tissue type based on one or more of an image or a portion of light energy reflected from the target tissue.

The laser system further includes a controller which is configured to control an output of the first laser light and the second laser light based on the target tissue type.

The controller is configured to select the first laser light or the second laser light based on a peak optical absorption of the target tissue.

The controller may be configured to output the first laser energy or the second laser energy based on a continuous feedback or a periodic feedback of the target tissue type.

The laser system may further include a rotating mirror which may be configured to receive the first laser light from the first laser and the second laser light from the second laser and direct at least one of the first laser light or the second laser toward an output of the medical laser system.

The first laser may include a Thulium-doped fiber, and wherein the second laser may include a YAG laser rod doped with Holmium.

The laser system may further include at least one actuator, wherein actuation of the at least one actuator may be configured to activate at least one of the first laser or the second laser.

The laser fiber may be only one laser fiber coupled to a single output of the laser system.

The first wavelength may be approximately 1940 nm and the second wavelength may be approximately 2120 nm.

According to another aspect, a method for performing a medical procedure using a laser system includes determining a first characteristic of a target tissue, selecting a first laser or a second laser based on the first characteristic of the target tissue, generating a first laser energy having a first wavelength from the first laser or a second laser energy having a second wavelength from the second laser based on the selection of the first laser or the second laser, wherein the first wavelength is different from the second wavelength.

The first wavelength may be approximately 1940 nm and the second wavelength may be approximately 2010 nm, and wherein the first characteristic of the target tissue may include a peak optical absorption of the target tissue.

The method may further include determining a second characteristic of the target tissue after generating at least one of the first laser and the second laser and generating the other of the first laser energy or the second laser energy if a value of the second characteristic is different from a value of the first characteristic.

According to another aspect, a method for performing a medical procedure using a laser system having a first laser configured to generate a first laser energy having a first wavelength, a second laser configured to generate a second laser energy having a second wavelength, a third laser configured to generate a third laser energy having a third wavelength, wherein the first, second, and third wavelengths are each different, and a laser fiber, wherein the method includes generating the first laser energy having the first wavelength, the second laser energy having the second wavelength, or the third laser energy having the third wavelength, emitting the first laser energy, the second laser energy, or the third laser energy from the laser fiber at a target tissue, receiving a feedback from the target tissue, determining a characteristic of the target tissue based on the feedback, selecting one of the first laser energy, the second laser energy, or the third laser energy based on the characteristic of the target tissue, and delivering the first laser energy, the second laser energy, or the third laser energy through the fiber based on the selection.

The characteristic may include a peak optical absorption of the target tissue, and the method may further include selecting a different one of the first laser energy, the second laser energy, or the third laser energy when a value of the peak optical absorption changes, and delivering the first laser energy, the second laser energy, or the third laser energy through the fiber based on the updated selection.

### BRIEF DESCRIPTION OF THE DRAWINGS

The accompanying drawings, which are incorporated in and constitute a part of this specification, illustrate various exemplary embodiments and together with the description, serve to explain the principles of the disclosed embodiments.
FIG. 1A is a schematic of a medical laser system according to an exemplary embodiment;
FIG. 1B is a schematic of a laser console of the medical laser system of FIG. 1A, according to an exemplary embodiment;
FIG. 2 is a schematic of a laser console of the medical laser system of FIG. 1A, according to another exemplary embodiment;
FIG. 3 is a schematic of a laser console of the medical laser system of FIG. 1A, according to another exemplary embodiment.

### DETAILED DESCRIPTION

This disclosure is described with reference to exemplary medical systems and medical tools for generating a laser energy for damaging a target site, for example, for ablating a target tissue (e.g., a stone) at a target site, by generating a laser energy from each of two or more laser generating devices, each laser energy having a different wavelength, and selectively outputting these laser energies at the target tissue, e.g., using a laser fiber. This may provide improved medical tool functionality and/or assist medical professionals to more easily and more quickly perform a medical procedure. However, it should be noted that reference to any particular device and/or any particular procedure is provided only for convenience and not intended to limit the disclosure. A person of ordinary skill in the art would recognize that the concepts underlying the disclosed devices and application methods may be utilized in any suitable procedure, medical or otherwise. This disclosure may be understood with reference to the following description and the appended drawings, wherein like elements are referred to with the same reference numerals.

For ease of description, portions of the disclosed devices and/or their components are referred to as proximal and distal portions. It should be noted that the term "proximal" is intended to refer to portions at an upstream end of a laser generation device, e.g., closer to a location of the generation of a laser energy, and the term "distal" is used herein to refer to portions downstream of the laser energy generation. Similarly, extends "distally" indicates that a component extends in a distal direction, and extends "proximally" indicates that a component extends in a proximal direction. Further, as used herein, the terms "about," "approximately" and "substantially" indicate a range of values within +/- 10% of a stated or implied value. Additionally, terms that indicate the geometric shape of a component/surface refer to exact and approximate shapes.

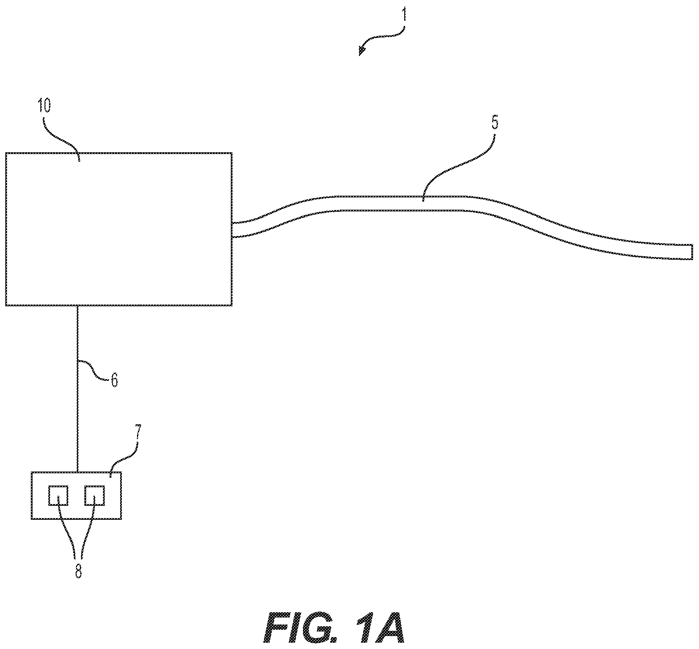

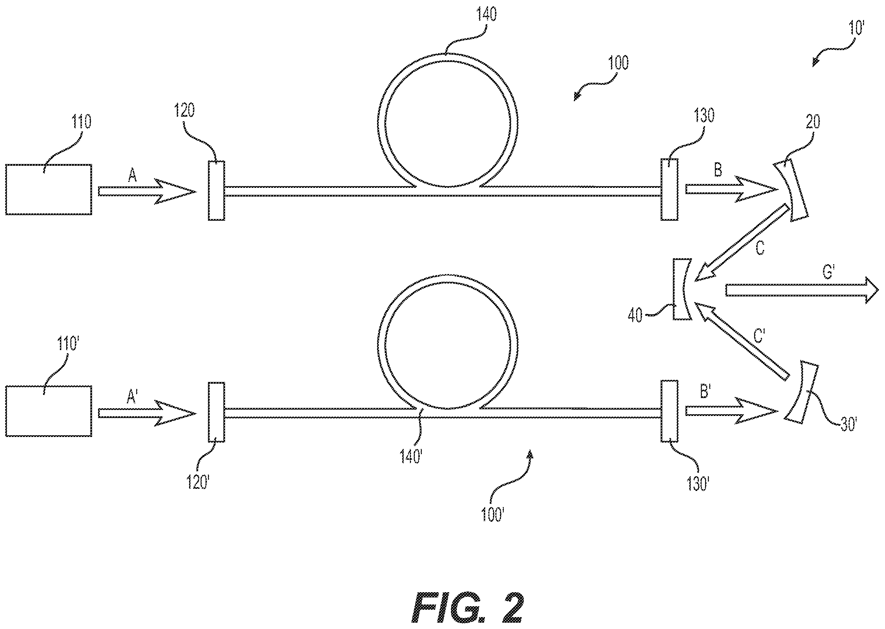

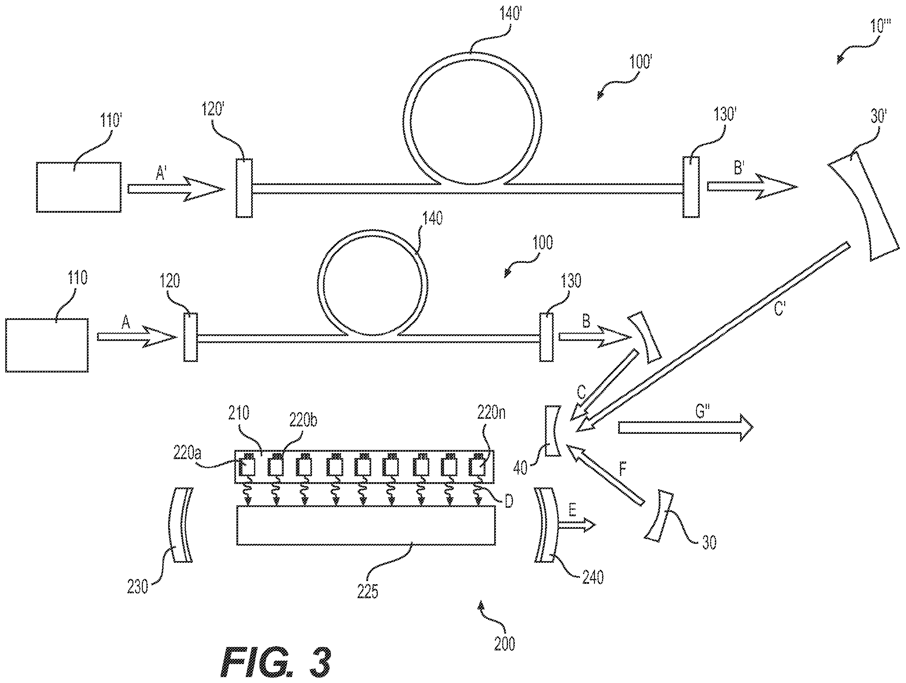

FIG. 1A illustrates an example of a medical laser system 1. Medical laser system 1 may include a medical laser console 10 for generating laser energy. The laser energy may be emitted toward a target tissue via a laser fiber 5 attached to medical laser console 10. A control device 7 having one or more actuators 8 may be attached to medical laser console 10. Control device 7 may include a processor and/or a display, or may be attached to a display. Actuators 8 may include a button, a switch, a foot pedal, or any other actuating device for controlling medical laser system 1 and/or laser generation by medical laser system 1. In some instances, additional user input devices may be attached to control device 7 and/or medical laser console 10, e.g., a graphical user interface (GUI), keyboard, etc. It will be understood that medical laser console 10 is an example console, and may include medical laser console 10' in FIG. 2 or medical laser console 10" in FIG. 3. In some embodiments, laser fiber 5 may be only one laser fiber 5 and may be attached to only one output. The only one output may output all laser energies generated by medical laser system 1, as described herein.

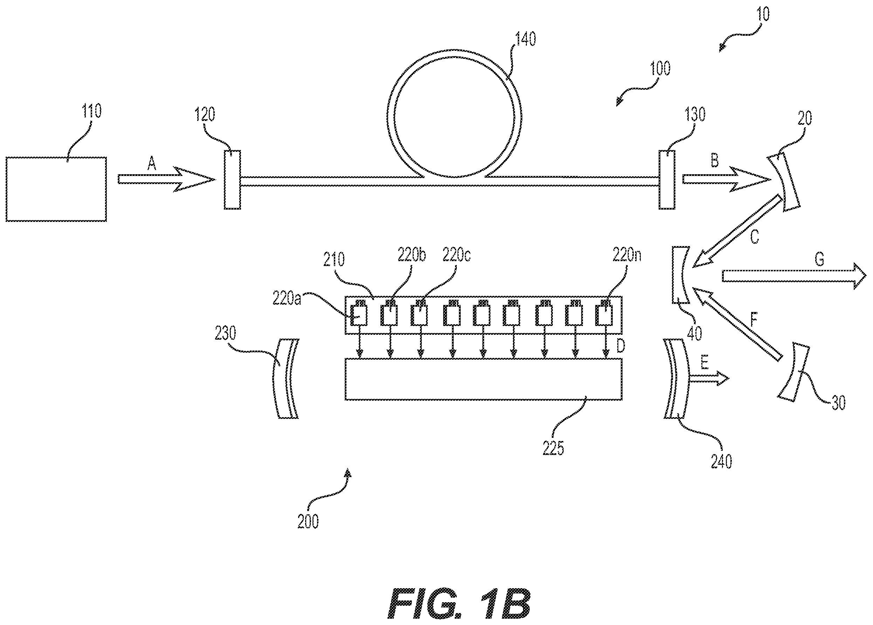

FIG. 1B illustrates an exemplary embodiment of a medical laser console 10. Medical laser console 10 includes a first laser generating device 100 and a second laser generating device 200. First laser generating device 100 may include a fiber laser for generating a first laser energy. One or more diode laser pumps 110 (only one diode laser pump shown in FIG. 1B) may generate a laser energy. The laser energy may be output toward a first mirror 120, e.g., a dielectric mirror, as shown by arrow A. A proximal facing surface of first mirror 120 may be transmissive to the laser energy, while a distal facing surface of first mirror 120 may be reflective of the laser energy. In this manner, the laser energy generated by laser pump 110 may pass through first mirror 120 only in a distal direction (as shown by arrow A) and not in a proximal direction, i.e., laser energy is prevented from passing through first mirror 120 in the proximal direction.

After passing through first mirror 120, the laser energy may subsequently pass through a gain fiber 140, which may provide light amplification and may change a wavelength of the laser energy. Gain fiber 140 may be a thulium-doped (Tm-doped) fiber. The grating and Tm-doping profile of gain fiber 140 may cause the laser energy generated by first laser generating device 100 to have a wavelength of approximately 1910 nm to approximately 1970 nm, approximately 1930 nm to approximately 1950 nm, or approximately 1940 nm. In some examples, the gratings of gain fiber 140 may include different spatial periodicities which may cause gain fiber 140 to determine a wavelength of the laser energy. For example, a distance between the gratings (e.g., a grating period) may be selected such that laser energy having a certain wavelength, e.g., 1910 nm, may be reflected by gain fiber 140. It will be understood that the spatial periodicities of the gratings of gain fiber 140 may be changed based on a desired wavelength(s) of the laser energy. Additionally, or alternatively, the dopant (e.g., Tm) may be an atom that that assists electronic transitions at the desired wavelength. For example, photons may be emitted at a desired wavelength based on the dopant. A second mirror 130 (e.g., a partially reflective mirror) may be disposed at a distal end of gain fiber 140. Second mirror 130 may be reflect laser energy having any wavelength different from the intended wavelength, e.g., 1940 nm, back toward gain fiber 140. Second mirror 130 may be transmissive to laser energy having the selected wavelength (a first laser energy), e.g., 1940 nm, and may allow the first laser energy to pass through second mirror 130 in a distal direction, as indicated by arrow B.

The first laser energy may pass from second mirror 130 toward a mirror 20 (e.g., a relay mirror) along the path indicated by arrow B. The first laser energy may pass from mirror 20 along the path indicated by arrow C toward a rotating mirror 40 (e.g., a Galvo mirror). Mirror 40 may reflect the first laser energy along path G toward an outlet of medical laser console 10. The first laser energy may be coupled to laser fiber 5 at the outlet of medical laser console 10 (e.g., FIG. 1) and may be emitted from laser fiber 5 at a target site.

With continued reference to FIG. 1B, second laser generating device 200 may include a laser diode device 210 including a plurality of optical pumps 220a, 220b, 220c, . . . 220n (hereinafter optical pumps 220). Optical pumps 220 may each generate a laser energy and may output the laser energy toward a laser rod 225, as indicated by arrows D. Laser rod 225 may be an yttrium-aluminum-garnet (YAG)-doped laser rod. For example, laser rod 225 may be a YAG laser rod doped with holmium (Ho) and Tm, which may cause laser energy to be output at a wavelength of 2120 nm. Optical pumps 220 may be diode lasers, which may have higher efficiency and reliability than conventional flash lamps. Diode lasers may also operate at lower voltage than conventional flash lamps, which may allow reduce the power supply and necessary to operate medical laser system 1 and may reduce the size of medical laser system 1. Additionally, liquid cooling may not be required for diode and low-power laser rod lasers, which may further reduce a size and/or cost of medical laser system 1. In this manner, multiple laser consoles (e.g., first laser console 100 and second laser console 200) may be formed as a medical laser system 1. It will be understood, however, that laser rod 225 may be a Ho:YAG laser rod and may be excited using flash lamps (e.g., optical pumps 220 may be flash lamps). It will also be understood that laser rod 225 is not limited to a YAG laser rod, and may be a Tm-doped YAG crystal which may be optimized for generating laser energy at approximately 2010 nm.

Emitting laser energy from optical pumps 220 to laser rod 225 may cause laser rod 225 to generate a second laser energy having a wavelength of approximately 2070 nm to approximately 2170 nm, approximately 2100 nm to approximately 2140 nm, or approximately 2120 nm. Second laser energy may travel proximally and distally, e.g., between a first mirror 230 and a second mirror 240. First mirror 230 may be a reflective mirror and may cause the second laser energy to be reflected distally, back toward laser rod 225. Second mirror 240 may be transmissive to one or more wavelengths, e.g., a wavelength of approximately 2120 nm. In this manner, second laser energy having a wavelength of approximately 2120 nm may pass through second mirror 240 as shown by arrow E. Second laser energy may pass from second mirror 230 and may travel along a path indicated by arrow E to impinge on a mirror 30 (e.g., a relay mirror). Mirror 30 may be reflective and may cause the second laser energy to be reflected toward mirror 40 along the path indicated by arrow F. Mirror 40 may then direct the second laser energy along the output path indicated by arrow G, similar to the first laser energy of first laser generating device 100. The second laser may also be coupled to laser fiber 5 and may be emitted at the target site.

A method of operating medical laser system 1 will be described with reference to FIGS. 1A and 1B. A distal end of laser fiber 5 may be inserted into a body via an opening (e.g., a natural orifice, an incision, etc.) and advanced to a target site. Laser fiber 5 may be used with another device, e.g., a scope such as a colonoscope, a duodenoscope, etc., to advance laser fiber 5 to the target site. For example, the scope may include imaging, articulation, grasping, and/or other capabilities, which may assist in positioning laser fiber 5 at the target site.

Once at the target site, a type of tissue on which a medical procedure may be performed may be determined. For example, a medical procedure may be performed on a stone, e.g., a kidney stone or a bladder stone, and the type of tissue forming the stone may be determined. In some examples, characteristics of the stone, e.g., density, size, shape, color, etc., may be determined by an imaging device associated with laser fiber 5 and/or the scope. Alternatively, the characteristics of the stone may be determined based on feedback from the stone, e.g., light reflected from the stone. In some examples, this may include using a laser energy associated with an aiming laser to determine the characteristics of the stone. Alternatively, or additionally, the characteristics may be determined based on feedback from the laser energy emitted by laser fiber 5 generated by medical laser console 10. In other words, the laser energy generated by medical laser console 10 may be emitted from laser fiber 5 prior to determining the characteristics of the target tissue.

With continued reference to FIGS. 1A and 1B, laser energy may be generated by either first laser generating device 100 or second laser generating device 200. One or more of actuators 8 (e.g., a first actuator) may be actuated to cause laser energy to be generated by laser diode pump 110. The laser energy emitted from laser diode pump 110 may pass distally through first mirror 120 and into gain fiber 140, as indicated by arrow A. The laser energy may be converted to a wavelength by the properties of gain fiber 140 in the first laser energy. For example, the first laser energy may include a wavelength of approximately 1940 nm. The first laser energy having a wavelength of approximately 1940 nm may pass distally through second mirror 130, as indicated by arrow B, while second mirror 130 may reflect laser energy having a wavelength other than approximately 1940 nm.

Once the first laser energy having a wavelength of approximately 1940 nm passes through second mirror 130, mirror 20 may reflect the first laser energy along the path indicated by arrow C toward rotating mirror 40. Rotating mirror 40 may be rotated automatically by a controller (not shown) or similar control device to properly align rotating mirror 40 with mirror 20 upon actuation of first laser generating device 100. The controller may be any processor (e.g. field programmable gate array (FPGA) device or the like) which may be programmed with instructions to perform one or more methods for controlling medical laser system 1, as described herein. The first laser energy is reflected from rotating mirror 40 along the path indicated by arrow G and coupled to laser fiber 5 (FIG. 1A). In some instances, the first laser energy may pass through additional mirrors, filters, beam combiners, or the like before being coupled to laser fiber 5. The first laser energy having wavelength 1940 nm may then be emitted from the distal end of laser fiber 5 toward the target tissue.

As described above, the first laser energy, or a portion of the first laser energy, may be reflected from the target tissue and may be detected by a detector. The reflected laser energy may provide the characteristics of the target tissue, and may enable a user to determine a suitable laser energy for performing the medical procedure. For example, based on the feedback from the target tissue, medical laser system 1 may determine that the first laser energy generated by first laser generating device 100 is the most suitable laser energy for performing the medical procedure. In this instance, medical laser system 1 may cause first laser generating device 100 to continue generating the first laser energy to be emitted via laser fiber 5 toward the target tissue.

Alternatively, it may be determined that laser energy having a wavelength generated by second laser generating device 200, e.g., the second laser energy, may be more suitable for performing the medical procedure. In this instance, the user may activate one or more of actuators 8 (e.g., a second actuation device) to cause second laser generating device 200 to generate the second laser energy. Alternatively, a controller of medical laser system 1 may automatically cause second laser generating device 200 to generate the second laser energy. Once second laser generating device 200 is activated, laser energy may be generated by optical pumps 220 and emitted toward laser rod 225 as indicated by arrows D. Laser rod 225 may cause the laser energy to have a portion of which has a wavelength of approximately 2120 nm. The second laser energy having the wavelength of approximately 2120 nm may be passed distally through second mirror 240 as shown by arrow E. Laser energy not having a wavelength of approximately 2120 nm may be reflected with a laser cavity between first mirror 230 and second mirror 240.

The second laser energy may pass from second mirror 240 toward mirror 30. Similar to mirror 20, the second laser energy may be reflected toward rotating mirror 40. Rotating mirror 40 may be automatically aligned with mirror 30 based on a selection or activation of second laser generating device 200, as described with reference to actuation of first laser generating device 100. The second laser energy may then pass along the path indicated by arrow G, as described herein.

It will be understood that continuous and/or periodic feedback, e.g., via imaging, feedback from the target tissue, and/or any other feedback, may allow the user and/or medical laser system 1 to select the laser generating device most suitable for performing the medical procedure. For example, the target tissue may include various different types of tissue, and each tissue may include different characteristics. The user, or a controller associated with medical laser system 1, may cause first laser generating device 100 and second laser generating device 200 to alternatively generate laser energy, based on the characteristics of the target tissue. In this manner, medical laser system 1 may generate a laser energy most suitable for the target tissue, without the need for multiple laser generating systems. This may also alleviate the need to remove a laser fiber to insert other laser fibers into the body throughout the medical procedure for delivering different laser energies. While the method was described by activating first laser generating device 100 prior to second laser generating device 200, it will be understood that first laser generating device 100 and second laser generating device 200 may be activated in any order, e.g., activating second laser generating device 200 prior to first laser generating device 100.

A medical laser console 10' according to another example is shown in FIG. 2. Medical laser console 10' may include first laser generating device 100 from FIG. 1B and a third laser generating device 100'. Third laser generating device 100' may include features similar to those as first laser generating device 100. For example, third laser generating device 100' may include a fiber laser for generating a third laser energy. One or more diode laser pumps 110' (only one diode laser pump shown in FIG. 2) may generate a laser energy. The laser energy may be output toward a first mirror 120', e.g., a dielectric mirror, as shown by arrow A'. A proximal facing surface of first mirror 120' may be transmissive to the laser energy, while a distal facing surface of first mirror 120' may be reflective of the laser energy. In this manner, the laser energy generated by laser pump 110' may pass through first mirror 120' only in a distal direction and not in a proximal direction, i.e., laser energy is presented from passing through first mirror 120' in the proximal direction.

The laser energy may subsequently pass through a gain fiber 140', which may provide light amplification and determine a wavelength of the laser energy at a distal end of gain fiber 140'. Similar to gain fiber 140, gain fiber 140' may also be a thulium-doped (Tm-doped) fiber, having a grating and doping profile which may cause the laser energy generated by third laser generating device 100' to have a wavelength of approximately 1960 nm to approximately 2060 nm, approximately 1990 nm to approximately 2030 nm, or approximately 2010 nm. As described herein, in some examples, the gratings of gain fiber 140' may include different spatial periodicities which may cause gain fiber 140' to determine a wavelength of the laser energy. For example, a distance between the gratings (e.g., a grating period) may be selected such that laser energy having a certain wavelength, e.g., 1960 nm, may be reflected by gain fiber 140'. It will be understood that the spatial periodicities of the gratings of gain fiber 140' may be changed based on a desired wavelength(s) of the laser energy. Additionally, or alternatively, the dopant (e.g., Tm) may be an atom that that assists electronic transitions at the desired wavelength. For example, photons may be emitted at a desired wavelength based on the dopant. A second mirror 130' (e.g., a partially reflective mirror) may be disposed at a distal end of gain fiber 140'. Second mirror 130' may reflect laser energy having any wavelength different from the intended wavelength, e.g., 2010 nm, back toward gain fiber 140'. Second mirror 130' may be transmissive to laser energy having the selected wavelength (a third laser energy), e.g., 2010 nm, and may allow laser energy having the selected wavelength to pass through second mirror 130' and in a distal direction, as indicated by arrow B'.

The third laser energy may pass from second mirror 130' toward a mirror 30' (e.g., a relay mirror). The third laser energy may pass from mirror 30' along the path indicated by arrow C' and toward rotating mirror 40. Mirror 40 may reflect the first laser energy along path G' toward an outlet of medical laser system 1. The third laser energy may be coupled to laser fiber 5 at the outlet of medical laser console 10' and supplied to a target site via laser fiber 5.

A method of operating laser system 1 having medical laser console 10' will be described with reference to FIG. 2. Similar to the method described with reference to FIG. 1B, laser fiber 5 may be inserted into the body and advanced to the target tissue. In one example, characteristics of the target tissue are determined before medical laser console 10' is activated. Alternatively, medical laser console 10' may be activated to assist in determining the characteristics of the target tissue, e.g., based on reflected light, as described herein.

Activation of first laser generating device 100 may be performed as described above. Third laser generating device 100' may be activated in a similar manner as first laser generating device 100. For example, one or more actuators 8 may cause third laser generating device 100' to be actuated to cause laser energy to be generated by laser diode pump 110'. The laser energy emitted from laser diode pump 110' may pass distally through first mirror 120' and into gain fiber 140', as indicated by arrow A'. The laser energy may be converted to a wavelength by the properties of gain fiber 140' in the third laser energy. For example, the third laser energy may include a wavelength of approximately 2010 nm. The first laser energy having a wavelength of approximately 2010 nm may pass distally through second mirror 130', as indicated by arrow B', while second mirror 130' may reflect laser energy having a wavelength other than approximately 2010 nm.

Once the third laser energy having a wavelength of approximately 2010 nm passes through second mirror 130', mirror 30' may reflect the first laser energy along the path indicated by arrow C' toward rotating mirror 40. Rotating mirror 40 may be rotated automatically by a controller (not shown) or similar control device to properly align rotating mirror 40 with mirror 30' upon actuation of third laser generating device 100'. The first laser energy is reflected from rotating mirror 40 along the path indicated by arrow G and coupled to laser fiber 5 (FIG. 1A). In some instances, the third laser energy may pass through additional mirrors, filters, beam combiners, or the like before being coupled to laser fiber 5. The third laser energy having wavelength 2010 nm may then be emitted from the distal end of laser fiber 5 toward the target tissue.

As described herein, first laser generating device 100 and third laser generating device 100' may be activated in any order, and any number of times based on the type of tissue. Medical laser system 1 may receive continuous or periodic feedback throughout the medical procedure, which may allow medical laser console 10' to determine the most suitable laser energy for performing the medical procedure, thereby selecting first laser generating device 100 and third laser generating device 100' to generate the appropriate laser energy. For example, the target tissue may include various different types of tissue, and each tissue may include different characteristics. The user, or a controller associated with medical laser system 1, may cause first laser generating device 100 and third laser generating device 100' to alternatively generate laser energy, based on the characteristics of the target tissue. In this manner, medical laser system 1 may generate a laser energy most suitable for the target tissue, without the need for multiple laser generating systems. This may also alleviate the need to remove a laser fiber to insert other laser fibers into the body throughout the medical procedure for delivering different laser energies. While the method was described by activating first laser generating device 100 prior to third laser generating device 100', it will be understood that first laser generating device 100 and third laser generating device 100' may be activated in any order, e.g., activating third laser generating device 100' prior to first laser generating device 100.

A medical laser console 10" according to another example is shown in FIG. 3. Medical laser console 10" may include first laser generating device 100, second laser generating device 200, and third laser generating device 100'. Each of these laser generating devices 100, 200, and 100' may be arranged to output laser energy along a path indicated by arrow G". For example, the first laser energy generated by first laser generating device 100 may have a wavelength of approximately 1940 nm and may be coupled to laser fiber 5 by traveling along a path indicated by arrows B, C, and G". The second laser energy generated by second laser generating device 200 may have a wavelength of approximately 2120 nm and may be coupled to laser fiber 5 by traveling along a path indicated by arrows E, F, and G". The third laser energy generated by third laser generating device 100' may have a wavelength of approximately 2010 nm and may be coupled to laser fiber 5 by traveling along a path indicated by arrows B', C', and G". In this manner the user and/or medical laser system 1 may select one or more of first, second, and third laser energies to be emitted from laser fiber 5 using a same medical laser console 10". While laser generating devices 100, 200, and 100' are shown in offset positions in FIG. 3, it will be understood that this is for understanding only, and the position of these laser generating devices 100, 200, and 100' is not limited. Moreover, the number of these laser generating devices 100, 200, and/or 100' is not limited. For example, a plurality of each of these laser generating devices 100, 200, and/or 100' may be provide in medical laser console 10", and may be arranged in any manner. Furthermore, the wavelengths of laser energies described herein is merely for example, and the wavelengths generated by these laser generating devices 100, 200, and 100' are not limited to those examples.

A method of operating medical laser system 1 having medical laser console 10" is described with reference to FIG. 3. Laser fiber 5 may be inserted into a body opening and advanced to the target tissue in a manner similar to that described in the methods with reference to FIGS. 1B and 2. As also described, the type of tissue maybe be determined before or after activation of laser generating devices 100, 200, and 100'. Laser generating devices 100, 200, and 100' may be activated in any manner described herein, e.g., via actuators 8 in FIG. 1A, and may be activated in any order. In this manner, first, second, and/or third laser energies, each laser energy having a different wavelength, may be generated by medical laser console 10" and emitted at the target tissue.

As described herein, it will be understood that continuous and/or periodic feedback, e.g., via imaging, feedback from the target tissue, and/or any other feedback, may allow the user and/or medical laser system 1 to select the laser generating device most suitable for performing the medical procedure. For example, the target tissue may include various different types of tissue, and each tissue may include different characteristics. The user, or a controller associated with medical laser system 1, may cause first laser generating device 100, second laser generating device 200, and/or third laser generating device 100' to alternatively generate laser energy, based on the characteristics of the target tissue. In this manner, medical laser system 1 may generate a laser energy most suitable for the target tissue, without the need for multiple laser generating systems. This may also alleviate the need to remove a laser fiber to insert other laser fibers into the body throughout the medical procedure for delivering different laser energies. While the method was described by activating first laser generating device 100 prior to second laser generating device 200 or third laser generating device 100', it will be understood that first laser generating device 100, second laser generating device 200, and third laser generating device 100' may be activated in any order, e.g., activating second laser generating device 200 prior to first laser generating device 100 and third laser generating device 100', activating third laser generating device 100' prior to first laser generating device 100 and second laser generating device 200, etc.

It will be understood that reference is made to a number of laser generating devices and/or mirrors in the medical laser system 1. It will be understood that the devices are not limited to this number and may change according to the requirement of the medical laser system 1. Further, while reference is made to a medical/surgical laser system, the laser generating techniques described herein is not limited to a medical/surgical laser system and may be used with any laser system. For example, medical laser system 1 may be used for any medical treatment, e.g., surgical treatments within the body or outside the body. These treatments may include prostate treatments, renal treatments, dental treatments, or other medical treatments. It will also be understood that while laser energies having certain wavelengths are described herein, laser energy having any wavelength suitable for conducting a medical treatment, e.g., approximately 600 nm to approximately 2200 nm may be generated by one or more laser generating devices of medical laser system 1. Additionally, or alternatively, medical laser system 1 may be used for any medical treatment of a tumor, a stone or tissue ablation, or the like. For example, laser energies having green or blue light (e.g., laser energies having a wavelength of 532 nm or 440 nm, respectively) may be used in medical treatments of soft tissues, e.g., tumors that are highly vascular. Laser energies having wavelengths in the 1910 nm to 2120 nm range may be useful for treating calculi, e.g., stone ablation and/or cracking calculi in blood vessels. It will be understood that these are only examples. Medical laser system 1 may efficiently and effectively treat different tissue types without the need for switching laser systems throughout a medical procedure. In this manner, medical procedures, and risks, time, energy, and costs associated therewith, may be reduced

It will be apparent to those skilled in the art that various modifications and variations can be made to the disclosed device without departing from the scope of the disclosure. Other embodiments of the disclosure will be apparent to those skilled in the art from consideration of the specification and practice of the invention disclosed herein. It is intended that the specification and examples be considered as exemplary only, with a scope of the invention being indicated by the following claims.

## Claims

1. A laser system (1), comprising:
a first laser (100) configured to generate a laser light, wherein the first laser light includes a first wavelength;
a second laser (200) configured to generate a second laser light, wherein the second laser light includes a second wavelength different from the first wavelength;
a laser fiber (5) operatively coupled to each of the first laser and the second laser configured to emit at least one of the first laser light or the second laser light at a target site;
a detector configured to detect a target tissue at the target site; **characterized by**
a controller configured to select the first laser light or the second laser light based on a peak optical absorption of the detected target tissue at the target site.

2. The laser system according to claim 1, wherein the first laser includes a Thulium-doped fiber, and wherein the second laser includes a YAG laser rod co-doped with Holmium and Thulium.

3. The laser system according to any of the preceding claims, further comprising a third laser configured to generate a third laser light, wherein the third laser light includes a third wavelength different from the first wavelength and the second wavelength.

4. The laser system according to claim 3, wherein the third laser includes a Thulium-doped fiber.

5. The laser system according to claims 3 and 4, wherein the third wavelength is approximately 2120 nm.

6. The laser system according to any one of the preceding claims, wherein the detector is configured to detect a target tissue type based on one or more of an image or a portion of light energy reflected from the target tissue.

7. The laser system according to claim 1, wherein the controller is configured to output the first laser energy or the second laser energy based on a continuous feedback or a periodic feedback of the target tissue type.

8. The laser system according to any of the preceding claims, further including a rotating mirror configured to receive the first laser light from the first laser and the second laser light from the second laser and direct at least one of the first laser light or the second laser light toward an output of the medical laser system.

9. The laser system according to claim 1, wherein the first laser includes a Thulium-doped fiber, and wherein the second laser includes a YAG laser rod doped with Holmium.

10. The laser system according to any of the proceeding claims, further comprising at least one actuator, wherein actuation of the at least one actuator is configured to activate at least one of the first laser or the second laser.

11. The laser system according to any of the preceding claims, wherein the laser fiber is only one laser fiber coupled to a single output of the laser system.

12. The laser system according to any one of the preceding claims, wherein the first wavelength is approximately 1940 nm and the second wavelength is approximately 2120 nm.

## Patentansprüche

1. Lasersystem (1), umfassend:
einen ersten Laser (100), der dazu konfiguriert ist, ein Laserlicht zu generieren, wobei das erste Laserlicht eine erste Wellenlänge aufweist;
einen zweiten Laser (200), der dazu konfiguriert ist, ein zweites Laserlicht zu generieren, wobei das zweite Laserlicht eine zweite Wellenlänge aufweist, die sich von der ersten Wellenlänge unterscheidet;
eine Laserfaser (5), die mit jedem von dem ersten und dem zweiten Laser wirkverbunden und dazu konfiguriert ist, mindestens eines von dem ersten Laserlicht oder dem zweiten Laserlicht an einer Zielstelle zu emittieren;
einen Detektor, der dazu konfiguriert ist, ein Zielgewebe an der Zielstelle zu erkennen; **gekennzeichnet durch**
eine Steuerung, die dazu konfiguriert ist, das erste Laserlicht oder das zweite Laserlicht basierend auf einer optischen Spitzenabsorption des erkannten Zielgewebes an der Zielstelle auszuwählen.

2. Lasersystem nach Anspruch 1, wobei der erste Laser eine Thuliumdotierte Faser aufweist und wobei der zweite Laser einen mit Holmium und Thulium kodotierten YAG-Laserstab aufweist.

3. Lasersystem nach einem der vorhergehenden Ansprüche, des Weiteren umfassend einen dritten Laser, der dazu konfiguriert ist, ein drittes Laserlicht zu generieren, wobei das dritte Laserlicht eine dritte Wellenlänge aufweist, die sich von der ersten Wellenlänge und der zweiten Wellenlänge unterscheidet.

4. Lasersystem nach Anspruch 3, wobei der dritte Laser eine Thuliumdotierte Faser aufweist.

5. Lasersystem nach den Ansprüchen 3 und 4, wobei die dritte Wellenlänge etwa 2120 nm beträgt.

6. Lasersystem nach einem der vorhergehenden Ansprüche, wobei der Detektor dazu konfiguriert ist, einen Zielgewebetyp basierend auf einem oder mehreren von einem Bild oder einem Anteil der von dem Zielgewebe reflektierten Lichtenergie zu erkennen.

7. Lasersystem nach Anspruch 1, wobei die Steuerung dazu konfiguriert ist, die erste Laserenergie oder die zweite Laserenergie basierend auf einer kontinuierlichen Rückmeldung oder einer periodischen Rückmeldung des Zielgewebetyps auszugeben.

8. Lasersystem nach einem der vorhergehenden Ansprüche, des Weiteren aufweisend einen rotierenden Spiegel, der dazu konfiguriert ist, das erste Laserlicht von dem ersten Laser und das zweite Laserlicht von dem zweiten Laser zu empfangen und mindestens eines von dem ersten Laserlicht oder dem zweiten Laserlicht auf einen Ausgang des medizinischen Lasersystems zu richten.

9. Lasersystem nach Anspruch 1, wobei der erste Laser eine Thuliumdotierte Faser aufweist und wobei der zweite Laser einen mit Holmium dotierten YAG-Laserstab aufweist.

10. Lasersystem nach einem der vorhergehenden Ansprüche, des Weiteren umfassend mindestens einen Aktuator, wobei die Betätigung des mindestens einen Aktuators dazu konfiguriert ist, mindestens einen von dem ersten Laser oder dem zweiten Laser zu aktivieren.

11. Lasersystem nach einem der vorhergehenden Ansprüche, wobei die Laserfaser nur eine Laserfaser ist, die an einen einzigen Ausgang des Lasersystems gekoppelt ist.

12. Lasersystem nach einem der vorhergehenden Ansprüche, wobei die erste Wellenlänge etwa 1940 nm und die zweite Wellenlänge etwa 2120 nm beträgt.

## Revendications

1. Système à lasers (1), comprenant :
un premier laser (100) configuré pour générer une première lumière laser, dans lequel la première lumière laser inclut une première longueur d'onde ;
un deuxième laser (200) configuré pour générer une deuxième lumière laser, dans lequel la deuxième lumière laser inclut une deuxième longueur d'onde différente de la première longueur d'onde ;
une fibre de laser (5) couplée de manière opérationnelle à chaque laser parmi le premier laser et le deuxième laser et configurée pour émettre au moins une lumière laser parmi la première lumière laser et la deuxième lumière laser au niveau d'un site cible ;
un détecteur configuré pour détecter un tissu cible au niveau du site cible ; **caractérisé par**
un contrôleur configuré pour sélectionner la première lumière laser ou la deuxième lumière laser sur la base d'une absorption optique de crête du tissu cible détecté au niveau du site cible.

2. Système à lasers selon la revendication 1, dans lequel le premier laser inclut une fibre dopée au thulium et dans lequel le deuxième laser inclut un barreau laser à base de YAG qui est co-dopé à l'holmium et au thulium.

3. Système à lasers selon l'une quelconque des revendications précédentes, comprenant en outre un troisième laser configuré pour générer une troisième lumière laser, dans lequel la troisième lumière laser inclut une troisième longueur d'onde différente de la première longueur d'onde et de la deuxième longueur d'onde.

4. Système à lasers selon la revendication 3, dans lequel le troisième laser inclut une fibre dopée au thulium.

5. Système à lasers selon les revendications 3 et 4, dans lequel la troisième longueur d'onde est d'approximativement 2120 nm.

6. Système à lasers selon l'une quelconque des revendications précédentes, dans lequel le détecteur est configuré pour détecter un type de tissu cible sur la base d'un ou de plusieurs moyens parmi une image et une partie d'énergie lumineuse qui est réfléchie depuis le tissu cible.

7. Système à lasers selon la revendication 1, dans lequel le contrôleur est configuré pour émettre en sortie la première énergie laser ou la seconde énergie laser sur la base d'un retour continu ou d'un retour périodique du type de tissu cible.

8. Système à lasers selon l'une quelconque des revendications précédentes, incluant en outre un miroir rotatif configuré pour recevoir la première lumière laser en provenance du premier laser et la deuxième lumière laser en provenance du deuxième laser et pour diriger au moins une lumière laser parmi la première lumière laser et la deuxième lumière laser en direction d'une sortie du système médical à lasers.

9. Système à lasers selon la revendication 1, dans lequel le premier laser inclut une fibre dopée au thulium et dans lequel le deuxième laser inclut un barreau laser à base de YAG dopé à l'holmium.

10. Système à lasers selon l'une quelconque des revendications précédentes, comprenant en outre au moins un actionneur, dans lequel l'actionnement de l'au moins un actionneur est configuré pour activer au moins un laser parmi le premier laser et le deuxième laser.

11. Système à lasers selon l'une quelconque des revendications précédentes, dans lequel la fibre de laser est seulement une seule fibre de laser qui est couplée à une unique sortie du système à lasers.

12. Système à lasers selon l'une quelconque des revendications précédentes, dans lequel la première longueur d'onde est d'approximativement 1940 nm et la deuxième longueur d'onde est d'approximativement 2120 nm.
